(19) Europäisches Patentamt
European Patent Office
Office européen des brevets

(11) Publication number: **0 315 827 B1**

(12) # EUROPEAN PATENT SPECIFICATION

(45) Date of publication of patent specification: **08.04.92**

(51) Int. Cl.5: **C07D 513/04**, A61K 31/47

(21) Application number: **88117809.9**

(22) Date of filing: **26.10.88**

The file contains technical information submitted after the application was filed and not included in this specification

(54) **Derivatives of quinolinecarboxylic acid.**

(30) Priority: **07.11.87 JP 281551/87**

(43) Date of publication of application:
**17.05.89 Bulletin 89/20**

(45) Publication of the grant of the patent:
**08.04.92 Bulletin 92/15**

(84) Designated Contracting States:
**BE CH DE ES FR GB IT LI NL SE**

(56) References cited:
**EP-A- 0 058 392**
**EP-A- 0 201 829**
**EP-A- 0 315 828**
**ZA-A- 8 703 179**

(73) Proprietor: **NIPPON SHINYAKU COMPANY, LIMITED**
**14, Kisshoin Nishinosho Monguchicho**
**Minami-ku Kyoto-shi Kyoto 601(JP)**

(72) Inventor: **Kise, Masahiro**
**Higashiiru Tominokoji Anayekoji-dori**
**Nakakyo-ku Kyoto 604(JP)**
Inventor: **Kitano, Masahiko**
**9-4 Donouecho Matsugasaki**
**Sakyo-ku Kyoto 606(JP)**
Inventor: **Ozaki, Masakuni**
**7-25 Mitani Terada**
**Joyo City 610-01(JP)**
Inventor: **Kazuno, Kenji**
**151-16 Kohirai**
**Rittocho 520-30(JP)**
Inventor: **Matsuda, Masahito**
**19-16 Seifucho**
**Otsu City 520-02(JP)**
Inventor: **Shirahase, Ichiro Nippon Shinyaku Yamashina Dormitory 39 Sakanotsujicho**
**Oyake Yamashinaku Kyoto 607(JP)**
Inventor: **Tomii, Yoshifumi**
**19-10 Hoshida-1-chome**
**Katano City 576(JP)**

(74) Representative: **Hranitzky, Wilhelm Max et al**
**NOVAPAT- CABINET CHEREAU 9, rue du Valais**
**CH-1202 Genève(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel derivatives of quinolinecarboxylic acid which exhibit antibacterial activity and are useful as therapeutic agents for various infectious diseases. More particularly, it relates to derivatives of quinolinecarboxylic acid represented by the following general formula (I) and physiologically-acceptable salt thereof.

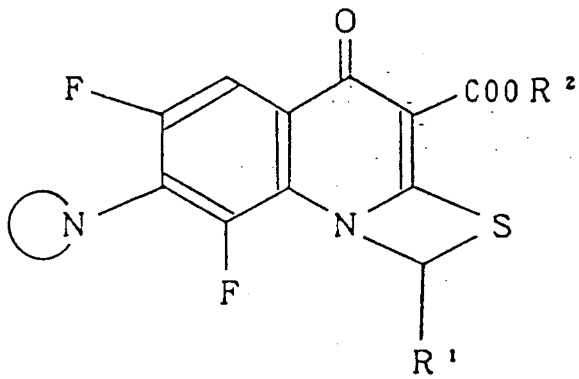

( I )

in which $R^1$ is hydrogen, straight chain or branched alkyl having 1 to 4 carbon atoms, unsubstituted phenyl or halogen-substituted phenyl; $R^2$ is hydrogen or straight chain or branched alkyl having 1 to 4 carbon atoms; and

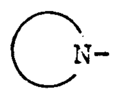

is five to six membered cyclic amino group which may be substituted with alkyl having 1 to 4 carbon atoms, amino or amino substituted with alkyl having 1 to 4 carbon atoms and may further contain nitrogen, oxygen or sulfur atom.

(Prior Art)

Nalidixic acid, piromidic acid, pipemidic acid, enoxacin (AT-2266), ofloxacin (DL-8280), have been widely used as synthetic antibacterials for therapy of infections by gram-negative bacteria. However they are not satisfactory for therapy of infectious diseases by gram-positive bacteria and chronic infectious diseases by Pseudomonas aeruginosa which have been increasing in recent years and are hard to be cured. In order to overcome the problem, various compounds have been synthesized and many patent applications have been filed.

The present inventors have synthesized various compounds too, found quinolinecarboxylic acids having excellent antibacterial activity, and filed a patent application already (Japanese Patent Application No. 79993/1987).

In said application paper, there is an actual disclosure on thiazetoquinolinecarboxylic acids in which 8-position is chlorine or bromine atom though there is no specific disclosure as to thiazetoquinolinecarboxylic acids in which fluorine atom is present at 8-position represented by the formula (I) in the present invention.

Thiazetoquinolinecarboxylic acid derivatives having a general formula similar to the above-indicated formula (I) are disclosed in European Patent Application No. 249 043.

However, this reference does not mention that thiazetoquinolinecarboxylic acid derivatives having fluorine both at 6- and 8- positions have advantageous properties distinguishing them from the other compounds having the disclosed general formula.

European Patent Application No. 201 829 discloses 1-aryl-4-quinoline-3-carboxylic acid derivatives. Such compounds differ from those of the present invention in that they do not have any thiazetidine ring.

Copending European Patent Application No. 315 828 of the same applicants as those of the present application discloses thiazetoquinolinecarboxylic acid derivatives having a formula similar to the formula (I) of the compounds of the present invention but with the 7-position of the quinoline moiety substituted with N-(5-methyl-2-oxo-1,3-dioxolen-4-yl) methyl.

European Patent Application No. 58 392 discloses a method of manufacture of chemical compounds and said method can be used for manufacturing some starting compounds for the obtention of the compounds of the present invention.

(Problems to be solved by the Invention)

There is certain limit as to the effect in the conventional antibacterials and their safety is not always satisfactory.

During the course of studies in order to overcome such difficulty, the present inventors have found a series of compounds which exhibit far better pharmacological activity than the above-given ones and exhibit lower toxicity and achieved the present invention.

Accordingly, an object of the present invention is to develop novel pharmaceuticals which exhibit far better properties than the above-given known synthetic antibacterials.

(Means to solve the Problems)

The present invention compounds are novel and have not been disclosed in the prior art literatures yet and their characteristic feature in terms of chemical structure is in the following two points.

1. A ring formed between nitrogen atoms and sulfur atom of the 2-mercaptoquinolone skeleton is thiazetidine ring; and
2. The quinoline skeleton is substituted with fluorine and cyclic amine at 8- and 7-positions, respectively.

As to alkyl represented by $R^1$ and $R^2$ in the general formula (I), lower alkyl in either straight or branched having 1 to 4 carbon atoms is preferred and is, for example, methyl, ethyl, n-propyl, isopropyl, n-butyl, isobutyl, sec-butyl, tert-butyl.

Examples of the substituents for phenyl represented by $R^1$ are one to several halogen atoms such as, for example, fluorine, chlorine, bromine, iodine, and, among those, fluorine is particularly preferred.

The cyclic amino group represented by

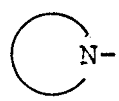

may be five to six membered one which may further contain nitrogen, oxygen or sulfur atom. Examples of such cyclic amino groups are pyrrolidino, piperidino, piperazino, morpholino, thiomorpholino.

Examples of substituents for such cyclic amino groups are alkyl or amino optionally substituted with alkyl. Examples of such alkyl are those exemplified for $R^1$ and $R^2$ hereinabove.

Examples of salts of the compound (I) of the present invention are salts with mineral acids such as hydrochloric acid, sulfuric acid, nitric acid, phosphoric acid, hydrofluoric acid, hydrobromic acid, salts with organic acids such as oxalic acid, acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, camphorsulfonic acid, and salts with alkali metals or alkali earth metals such as sodium, potassium, calcium.

Compounds of the present invention may, for example, be prepared in accordance with the following manner.

<u>Method A</u>

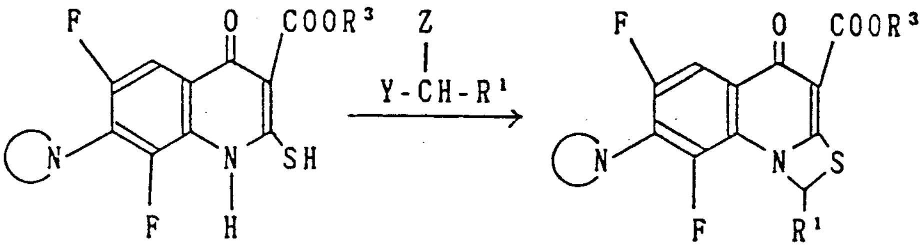

( II ) ( I a )

in which $R^1$ and

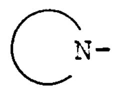

are the same as those defined already; Y and Z are same or different and stand for halogen; and $R^3$ is alkyl.

<u>Method B</u>

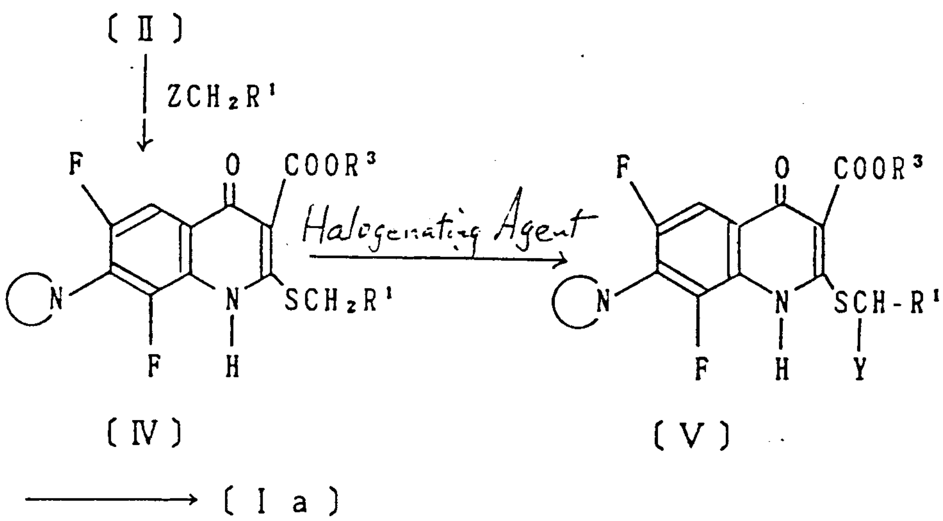


( II ) ( IV ) ( V ) ( I a )

in which $R^1$, $R^3$,

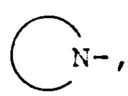

Y and Z are the same as those in Method A.

<u>Method C</u>

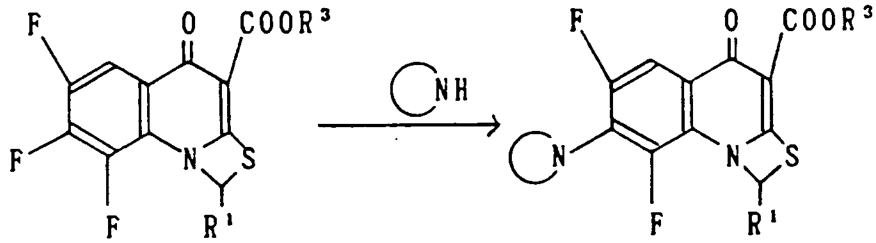

(VI) (Ia)

in which $R^1$, $R^3$ and

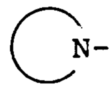

are the same as those in Method A.

Method D

(VI)

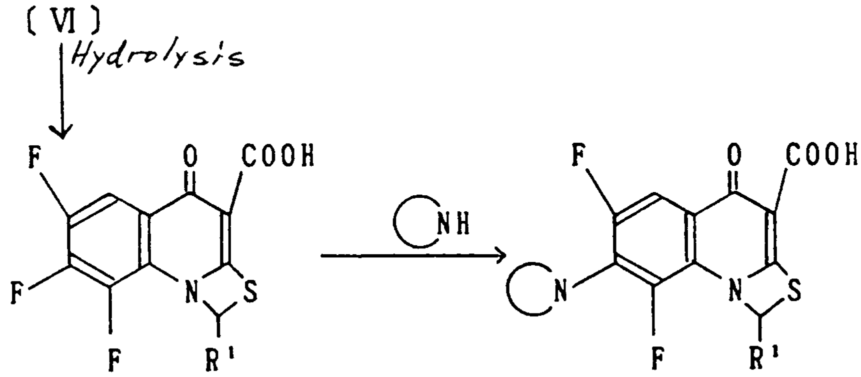


(VII) (Ib)

in which $R^1$ and

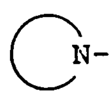

are the same as already defined.

As will be obvious from the above, the compounds of the present invention can be manufactured in two approaches.

One of them is that quinolinecarboxylic acid in which 7-position is substituted with cyclic amino group is used as a starting material and thiazetidine ring is produced therefrom (Methods A and B) while another is that, after formation of thiazetidine ring, cyclic amino group is introduced into 7-position (Methods C and D). Each method will be further illustrated hereinafter.

Method A: (II) is made to react with dihalide (e.g. methylene iodide, ethylidene bromide or benzylidene bromide) in an inert solvent in the presence of acid removing agent (e.g. sodium carbonate, potassium carbonate or triethylamine) usually at 0 to 120 °C so that cyclization is carried out to give (Ia).

As to a solvent, aprotic ones such as N,N-dimethylformamide, N,N-dimethylacetamide, dimethyl sulfoxide or sulforan). The amount of the dihalide and acid-removing agent is not less than equimolar to one mole of (II) and is preferably 1.1 to 2.5 moles. In order to accelerate the reaction, 0.01 to 3.0 molar

equivalents of sodium iodide or potassium iodide may be added to the reaction system.

Method B: (II) is made to react with a halide ($ZCH_2R^1$) using the same solvent and acid-removing agent as in the method A usually at 0 to 80 ° C to manufacture (IV).

Then (IV) is halogenated in an inert solvent (e.g. halogenated hydrocarbon type solvent such as chloroform, dichloromethane or carbon tetrachloride) using a halogenating agent (e.g. N-bromosuccinimide or N-chlorosuccinimide) to manufacture (V). (V) is then cyclized using the same solvent and acid removing agent as in method A usually at 0 to 80 ° C to give (Ia).

Method C: Condensation of (VI) with cyclic amine gives (Ia). This reaction is conducted in an inert solvent (e.g. aprotic solvent such as N,N-dimethylformamide, N,N-dimethylacetamide, dimethyl sulfoxide, sulforan or acetonitrile) preferably in the presence of an acid removing agent (e.g. sodium carbonate, potassium carbonate, sodium bicarbonate, potassium bicarbonate or triethylamine) usually at 0 to 80 ° C, for example at 40 to 60 ° C, using a cyclic amine as a reactant. The amount of the cyclic amine used is 1.5 to 2.5 moles to one mole of (VI).

Method D: (VI) is hydrolyzed with an acid (e.g. concentrated sulfuric acid, fuming sulfuric acid, polyphosphoric acid or a mixture thereof) to give (VII). This reaction is conducted using an excess of acid as a solvent (one to 30 times as much by weight or, preferably, 5 to 10 times as much) usually at 0 to 60 ° C. This hydrolyzing reaction may also be conducted in 20 to 30 times as much (preferably 5 to 10 times as much) 1 to 5% solution of potassium hydroxide or sodium hydroxide in aqueous alcohol (e.g. methanol, ethanol, propanol or butanol) usually at the temperature range of from room temperature to 60 ° C.

Then (VII) is made to react with cyclic amine in the same solvent as in method C to give (Ib). The reaction is usually conducted at 0 to 60 ° C though 0 ° C to room temperature is preferred.

Other methods will be that, as given below, a compound of the general formula (VIII) is used as a starting material.

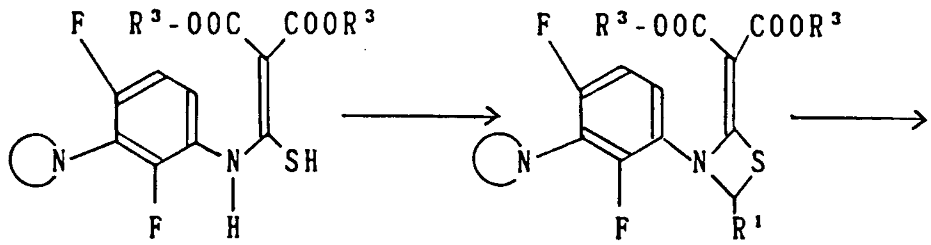

( VIII )  ( IX )

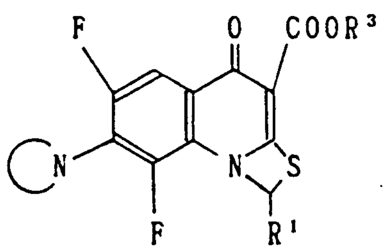

( I a )

wherein $R^1$, $R^3$ and

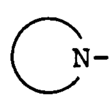

are the same as those defined in the method A.

Thus (VIII) is made to react with dihalide in the presence of an acid removing agent (e.g. potassium

carbonate) in an inert solvent (e.g. N,N-dimethylformamide). Then (IX) is subjected to a ring closure to give (Ia). This ring closure reaction may be carried out by a method known per se such as, for example, by a method with heating or by a method using acidic substance such as phosphorus oxychloride, phosphorus pentachloride, phosphorus trichloride, thionyl chloride, fuming sulfuric acid, concentrated sulfuric acid, polyphosphoric acid or polyphosphate. When an acid substance is used for example, one mole to great excess (preferably 20 to 30 moles) of acidic substance per mole of (IX) is used and the reaction temperature is usually 0 to 100 ° C or, preferably, from 0 to 60 ° C.

Another method will be that thiazetidine ring is produced using 2,3,4-trifluoro compounds as a starting material, subjected to a ring closure and condensed with an amine by the same manner as in method C to give (Ia).

When diamine such as piperazine is made to react in the above manufacturing method, one nitrogen atom thereof may, if necessary, be protected by a method known per se and made to react with (VI) followed by detaching the protective group therefrom to give a desired compound having no substituent at nitrogen.

Moreover, a substituent may be introduced into nitrogen atom to the N-unsubstituted one by a known method per se to afford N-substituted diamino compound.

When the compounds prepared in accordance with the above method is an ester ($R^2$ is alkyl), it may, if and when desired, be hydrolyzed to give the corresponding carboxylic acid ($R^2$ is hydrogen). The hydrolysis can be conducted by the use of a great excess of acid (e.g. sulfuric acid, fuming sulfuric acid, hydrochloric acid, hydrobromic acid, hydrobromic acid/acetic acid, chlorosulfonic acid or polyphosphoric acid), preferably 10 to 20 times as much acid, as a solvent at the temperature of from room temperature to 110 ° C. Alternatively, the hydrolysis may be conducted by stirring at the temperature of from room temperature to 60 ° C in 2 to 30 times as much volume (preferably 5 to 10 times as much volume) of 1 to 5% solution of potassium hydroxide or sodium hydroxide in aqueous alcohol such as methanol, ethanol, propanol or butanol (preferably, tert-butanol).

Further, the ester may be heated at 60-150 ° C, preferably at 100-110 ° C, with stirring in 10 to 100 times as much amount of alcohol corresponding to the desired ester in the presence of a catalytic amount of concentrated sulfuric acid so that the ester can be converted to desired another ester.

In the case of a carboxylic acid (i.e. $R^2$ is hydrogen), it can, if and when desired, be esterified to give desired ester (i.e. $R^2$ is alkyl). This esterification reaction can be conducted by a method known per se such as, for example, by the use of thionyl chloride with alcohol, condensing agent (e.g. dicyclocarbodiimide) with alcohol, or alkyl halide with alcoholate. Furthermore, in the case of a carboxylic acid, it can be used in a form of pharmacologically-acceptable salt such as sodium or potassium salt.

Both starting materials (II) and (VIII) are novel and such novel compounds can be manufactured by a known method (e.g. by a method disclosed in European Patent Application EP-A-58 392).

Novel starting material (VI) will be disclosed later in reference examples and may be manufactured in accordance with the above methods A and B. Cyclic amines are known substances and can be manufactured by a known method.

The prepared compound (I) as such can be isolated and purified by a known method per se such as, for example, concentration, pH conversion, transfer to another solvent, extraction with a solvent, crystallization, recrystallization, fractional distillation or chromatography.

When the compound of the present invention is administered as a pharmaceutical drug, it is given to animals including human being in a form of a pharmaceutical composition containing 0.1 to 99.5%, preferably 0.5 to 90%, of the compound invention in a pharmaceutically-acceptable, nontoxic and inert carrier or is given to them as it is without a carrier.

Examples of the carrier applicable are one or more solid, semisolid or liquid diluent, filler or other auxiliary agent for pharmaceutical prescription. It is desired that the pharmaceutical composition is administered in a unit dose form. The pharmaceutical composition of the present invention can be administered via mouth, tissue, local place (e.g. skin) or rectum. Needless to say, the pharmaceutical form is selected suitably for each administration route. Oral administration is, for example, especially preferred.

Dose as a remedy for infectious diseases is desirably adjusted by taking the conditions of the patient (e.g. age or body weight), administration route, and type and degree of the disease into consideration but, usually, it is within a range of 50 to 1000 mg of the present invention compound/human being/day and, preferably, 100 to 300 mg. In some cases, less dose may be sufficient while in other case, more dose may be necessary. It is desired to administer dividedly, i.e. 2 or 3 times a day.

(Examples)

The present invention will be further illustrated by way of the reference and working examples relating to the manufacture of the present invention compounds as well as test examples for the present invention compounds.

Reference Examples.

(1) Thiophosgene (1.4 g) was dissolved in 25 ml of ether, 2.0 g of triethylamine was added thereto with ice cooling and stirring (at not higher than 20° C), then a mixture of 1.4 g of 2,3,4-trifluoroaniline and 3 ml of ether was gradually dropped thereinto at the same temperature, and the mixture was stirred for one hour at the same temperature. Then it was filtered off, the filtrate was concentrated, the residue was extracted with n-hexane, insoluble matters were removed by filtration, the resulting hexane-soluble matters were subjected to a silica gel columm chromatography (Wacogel C-200 in the amount of 15 g) and eluted with n-hexane to give 1.39 g of 2,3,4-trifluorophenyl isothiocyanate.

(2) Into 90 ml of dry tetrahydrofuran was suspended 2.0 g of 60% sodium hydride, 8.0 g of diethyl malonate was dropped thereinto with stirring at room temperature, the mixture was stirred for one hour at the same temperature, a solution of 9.0 g of the compound obtained in (1) in 5 ml of dry tetrahydrofuran was dropped thereinto with stirring at room temperature and, one hour thereafter, 1.0 g of triethylamine was added, then 4.2 g of chloromethyl methyl ether was dropped thereinto, and the mixture was stirred for one hour. The reaction solution was poured over into ice water, extracted with ethyl acetate, washed with 1% hydrochloric acid, aqueous solution of sodium bicarbonate and aqueous solution of sodium chloride succesively, and dried over anhydrous magnesium sulfate. Ethyl acetate was evaporated therefrom to give 18.3 g of diethyl 2,3,4-trifluorophenylaminomethoxymethylthiomethylenemalonate.

(3) The compound obtained in (2) (18 g) was added to 100 ml of diphenyl ether and the mixture was stirred for 15 minutes at 230° C with removal of ethanol therefrom in vacuo. After cooling, it was subjected to a silica gel column chromatography (300 g of Wakogel C-300 being used), diphenyl ether was eluted out with n-hexane and then eluted with chloroform to give 10.62 g of ethyl 6,7,8-trifluoro-4-hydroxy-2-methoxymethylthioquinoline-3-carboxylate.

(4) The compound obtained in (3) (10.5 g) was suspended in 60 ml of ethanol, 60 ml of concentrated hydrochloric acid was dropped thereinto, and the mixture was stirred at 50° C for 1.5 hours. The reaction solution was poured over ice water, the crystals separated out therefrom were collected by filtration, washed with water, air-dried, and washed with n-hexane to give 6.8 g of ethyl 6,7,8-trifluoro-4-hydroxy-2-mercaptoquinoline-3-carboxylate.

(5) To 25 ml of N,N-dimethylformamide were added 12 g of diiodoethane and 8.3 g of potassium carbonate, a solution of 6.07 g of the compound obtained in (4) dissolved in 60 ml of N,N-dimethylformamide was gradually dropped thereinto, and the mixture was stirred for 30 minutes at the same temperature. N,N-Dimethylformamide was evaporated therefrom in vacuo, the residue was dissolved in a mixture of chloroform and methanol (2:1), washed with water, dried over magnesium sulfate, the solvent was evaporated therefrom, and the crystals separated out therefrom were washed with ether to give 3.35 g of ethyl 6,7,8-trifluoro-1-methyl-4-oxo-4H - [1,3]thiazeto[3,2-a]quinoline-3-carboxylate.

Example 1. Ethyl 6,8-difluoro-1-methyl-4-oxo-7-(1-piperazinyl)-4H-[1,3]thiazeto[3,2-a]quinoline-3-carboxyate.

Ethyl 6,7,8-trifluoro-1-methyl-4-oxo-4H-[1,3]thiazeto-[3,2-a]quinoline-3-carboxylate (0.33 g) and 0.14 g of potassium carbonate were suspended in 10 ml of N,N-dimethylformamide, 0.13 g of anhydrous piperazine was added and the mixture was stirred at 60° C for 2 hours. N,N-Dimethylformamide was evaporated therefrom in vacuo, the residue was dissolved in chloroform, the solution was washed with water, dried over magnesium sulfate, the solvent was evaporated therefrom, the residue was subjected to a silica gel column chromatography (30 g of Wakogel C-300 being used) and eluted with chloroform-methanol (4:1) to give 0.29 g of the title product, m.p. 195-198° C (decompn).

| Elem. Anal. for $C_{18}H_{19}F_2N_3O_3S \cdot \frac{1}{4} H_2O$ | | | |
|---|---|---|---|
| Calcd (%):<br>Found (%): | C 54.06,<br>C 54.12, | H 4.91,<br>H 5.02, | N 10.51<br>N 10.35 |

Example 2. 6,8-difluoro-1-methyl-4-oxo-7-(1-piperazinyl)-4H-[1,3]thiazeto[3,2-a]quinoline-3-carboxylic acid.

To 0.5 g of ethyl 6,8-difluoro-1-methyl-4-oxo-7-(1-piperazinyl)-4H-[1,3]thiazeto[3,2-a]quinoline-3-carboxylate was added 10 ml of 5% solution of potassium hydroxide in a 3:1 mixture of tert-butanol and water and

the mixture was stirred at 60° C for 30 minutes. tert-Butanol was evaporated therefrom in vacuo and the residue was neutralized with acetic acid. After cooling, the crystals separated out therefrom were collected by filtration, washed with water and then washed with acetone and ether to give 0.43 g of the title product, m.p. 260° C (decompn.).

| Elem. Anal. for $C_{16}H_{15}F_2N_3O_3S.\frac{3}{4}H_2O$ | | | |
|---|---|---|---|
| Calcd (%): | C 50.46, | H 4.37, | N 11.03 |
| Found (%): | C 50.29, | H 4.54, | N 10.97 |

Example 3. Ethyl 6,8-difluoro-1-methyl-7-(4-methyl-1-piperazinyl)-4-oxo-4H-[1,3]thiazeto[3,2-a]quinoline-3-carboxylate.

Ethyl 6,7,8-trifluoro-1-methyl-4-oxo-4H-[1,3]thiazeto-[3,2-a]quinoline-3-carboxylate (1.05 g) and 0.44 g of potassium carbonate were suspended in 20 ml of N,N-dimethylformamide, 0.48 g of N-methylpiperazine was added and the mixture was stirred at 60° C for 6 hours. N,N-Dimethylformamide was evaporated therefrom in vacuo, the residue was dissolved in chloroform, the solution was washed with water, dried over magnesium sulfate, the solvent was evaporated therefrom, the residue was subjected to a silica gel column chromatography (50 g of Wakogel C-300 being used) and eluted with a 50:1 mixture of chloroform and methanol to give 0.79 g of the title product, m.p. 183-185° C.

| Elem. Anal. for $C_{19}H_{21}F_2N_3O_3S.\frac{1}{4}H_2O$ | | | |
|---|---|---|---|
| Calcd (%): | C 55.13, | H 5.23, | N 10.15 |
| Found (%): | C 54.99, | H 5.29, | N 10.12 |

Example 4. 6,8-Difluoro-1-methyl-7-(4-methyl-1-piperazinyl)-4-oxo-4H-[1,3]thiazeto[3,2-a]quinoline-3-carboxylic acid.

To 0.5 g of ethyl 6,8-difluoro-1-methyl-7-(4-methyl-1-piperazinyl)-4-oxo-4H-[1,3]thiazeto[3,2-a]quinoline-3-carboxylate was added 10 ml of 5% solution of potassium hydroxide in 3:1 mixture of tert-butanol and water and stirred at 60° C for 30 minutes. tert-Butanol was evaporated therefrom in vacuo, the residue was neutralized with acetic acid, extracted with a 3:1 mixture of chloroform and methanol, the extract was washed with saturated sodium chloride solution, dried over magnesium sulfate, the solvent was evaporated therefrom, and the residue was subjected to a silica gel column chromatography (30 g of Wakogel C-300 being used) and eluted with chloroform-methanol (10:1) to give 0.18 g of desired product, m.p. 238° C (decompn.).

| Elem. Anal. for $C_{17}H_{17}F_2N_3O_3S.\frac{1}{4}H_2O$ | | | |
|---|---|---|---|
| Calcd (%): | C 52.91, | H 4.57, | N 10.89 |
| Found (%): | C 52.93, | H 4.64, | N 10.95 |

Similarly prepared were the following compounds.

Example 5. 6,8-Difluoro-4-oxo-7-(1-piperazinyl)-4-oxo-4H-[1,3]thiazeto[3,2-a] quinoline-3-carboxylic acid.
Mass analysis ($C_{15}H_{13}F_2N_3O_3S$) $M^+$: 353

Example 6. Ethyl 6,8-difluoro-4-oxo-7-(1-piperazinyl)-4H- [1,3]thiazeto[3,2-a]quinoline-3-carboxylate.
Mass analysis ($C_{17}H_{17}F_2N_3O_3S$) $M^+$: 381.

Example 7. 6,8-Difluoro-7-(4-methyl-1-piperazinyl)-4-oxo-4H-[1,3]thiazeto[3,2-a]quinoline-3-carboxylic acid.
Mass analysis ($C_{16}H_{15}F_2N_3O_3S$) $M^+$: 367

Example 8. Ethyl 6,8-difluoro-7-(4-methyl-1-piperazinyl)-4-oxo-4H-[1,3]thiazeto[3,2-a]quinoline-3-carboxylate.
Mass analysis ($C_{18}H_{19}F_2N_3O_3S$) $M^+$: 395

Example 9. 7-(3-Amino-1-pyrrolidinyl)-6,8-difluoro-4-oxo-4H-[1,3]thiazeto[3,2-a]quinoline-3-carboxylic acid.
Mass analysis ($C_{15}H_{13}F_2N_3O_3S$) $M^+$: 353

Example 10. Ethyl 7-(3-amino-1-pyrrolidinyl)-6,8-difluoro-4-oxo-4H-[1,3]thiazeto[3,2-a]quinoline-3-carboxylate.

Mass analysis ($C_{17}H_{17}F_2N_3O_3S$) $M^+$: 381

Example 11. 6,8-Difluoro-7-(3-methylamino-1-pyrrolidinyl)-4-oxo-4H-[1,3]thiazeto[3,2-a]quinoline-3-carboxylic acid.

Mass analysis ($C_{16}H_{15}F_2N_3O_3S$) $M^+$: 367

Example 12. Ethyl 6,8-difluoro-7-(3-methylamino-1-pyrrolidinyl)-4-oxo-4H-[1,3]thiazeto[3,2-a]quinoline-3-carboxylate.

Mass analysis ($C_{18}H_{19}F_2N_3O_3S$) $M^+$: 395

Example 13. 6,8-Difluoro-7-(3-methyl-1-piperazinyl)-4-oxo-4H-[1,3]thiazeto[3,2-a]quinoline-3-carboxylic acid.

Mass analysis ($C_{16}H_{15}F_2N_3O_3S$) $M^+$ 367

Example 14. Ethyl 6,8-difluoro-7-(3-methyl-1-piperazinyl)-4-oxo-4H-[1,3]thiazeto[3,2-a]quinoline-3-carboxylate.

Mass analysis ($C_{18}H_{19}F_2N_3O_3S$) $M^+$: 395

Example 15. 6,8-Difluoro-7-(3,4-dimethyl-1-piperazinyl)-4-oxo-4H-[1,3]thiazeto[3,2-a]quinoline-3-carboxylic acid.

Mass analysis ($C_{17}H_{17}F_2N_3O_3S$) $M^+$: 381

Example 16. Ethyl 6,8-difluoro-7-(3,4-dimethyl-1-piperazinyl)-4-oxo-4H-[1,3]thiazeto[3,2-a]quinoline-3-carboxylate.

Mass analysis ($C_{19}H_{21}F_2N_3O_3S$) $M^+$: 409

Example 17. 6,8-Difluoro-7-thiomorpholino-4-oxo-4H-[1,3]thiazeto[3,2-a]quinoline-3-carboxylic acid.

Mass analysis ($C_{16}H_{14}F_2N_2O_3S_2$) $M^+$: 384

Example 18. Ethyl 6,8-difluoro-7-thiomorpholino-4-oxo-4H-[1,3]thiazeto[3,2-alquinoline-3-carboxylate.

Mass analysis ($C_{18}H_{18}F_2N_2O_3S_2$) $M^+$: 412

Example 19. 6,8-Difluoro-7-morpholino-4-oxo-4H-[1,3]thiazeto[3,2-a]quinoline-3-carboxylic acid.

Mass analysis ($C_{16}H_{14}F_2N_2O_4S$) $M^+$: 368

Example 20. Ethyl 6,8-difluoro-7-morpholino-4-oxo-4H-[1,3]thiazeto[3,2-a]quinoline-3-carboxylate.

Mass analysis ($C_{18}H_{18}F_2N_2O_4S$) $M^+$: 396

Example 21. 6,8-Difluoro-4-oxo-1-phenyl-7-(1-piperazinyl)-4H-[1,3]thiazeto[3,2-a]quinoline-3-carboxylic acid.

Mass analysis ($C_{21}H_{17}F_2N_3O_3S$) $M^+$: 429

Example 22. Ethyl 6,8-difluoro-4-oxo-1-phenyl-7-(1-piperazinyl)-4H-[1,3]thiazeto[3,2-a]quinoline-3-carboxylate.

Mass analysis ($C_{23}H_{21}F_2N_3O_3S$) $M^+$: 457

Example 23. 6,8-Difluoro-7-(4-methyl-1-piperazinyl)-4-oxo-1-phenyl-4H-[1,3]thiazeto[3,2-a]quinoline-3-carboxylic acid.

Mass analysis ($C_{22}H_{19}F_2N_3O_3S$) $M^+$: 443

Example 24. Ethyl 6,8-difluoro-7-(4-methyl-1-piperazinyl)-4-oxo-1-phenyl-4H-[1,3]thiazeto[3,2-a]-quinoline-3-carboxylate.

Mass analysis ($C_{24}H_{23}F_2N_3O_3S$) $M^+$: 471

Example 24a. 6,8-Difluoro-1-(4-fluorophenyl)-4-oxo-7-(1-piperazinyl)-4H-[1,3]thiazeto[3,2-a]quinoline-3-carboxylic acid.

Mass analysis ($C_{21}H_{16}F_3N_3O_3S$) $M^+$: 447

Example 25. Ethyl 6,8-difluoro-1-(4-fluorophenyl)-4-oxo-7-(1-piperazinyl)-4H-[1,3]thiazeto[3,2-a]quinoline-3-carboxylate.

Mass analysis ($C_{23}H_{20}F_3N_3O_3S$) $M^+$: 475

Example 26. 6,8-Difluoro-1-(4-fluorophenyl)-7-(4-methyl-1-piperazinyl)-4-oxo-4H-[1,3]thiazeto[3,2-a]-quinoline-3-carboxylic acid.

Mass analysis ($C_{22}H_{18}F_3N_3O_3S$) $M^+$: 461

Example 27. Ethyl 6,8-difluoro-1-(4-fluorophenyl)-7-(4-methyl-1-piperazinyl)-4-oxo-4H-[1,3]thiazeto[3,2-a]quinoline-3-carboxylate.

Mass analysis ($C_{24}H_{22}F_3N_3O_3S$) $M^+$: 489

Example 28. 6,8-Difluoro-1-(2,4-difluorophenyl)-4-oxo-7-(1-piperazinyl)-4H-[1,3]thiazeto[3,2-a]quinoline-3-carboxylic acid.

Mass analysis ($C_{21}H_{15}F_4N_3O_3S$) $M^+$: 465

Example 29. Ethyl 6,8-difluoro-1-(2,4-difluorophenyl)-4-oxo-7-(1-piperazinyl)-4H-[1,3]thiazeto[3,2-a]-quinoline-3-carboxylate.

Mass analysis ($C_{23}H_{19}F_4N_3O_3S$) $M^+$: 493

Example 30. 6,8-Difluoro-1-(2,4-difluorophenyl)-7-(4-methyl-1-piperazinyl)-4-oxo-4H-[1,3]thiazeto[3,2-a]-

quinoline-3-carboxylic acid.
Mass analysis ($C_{22}H_{17}F_4N_3O_3S$) $M^+$: 479

Example 31. Ethyl 6,8-difluoro-1-(2,4-difluorophenyl)-7-(4-methyl-1-piperazinyl)-4-oxo-4H-[1,3]thiazeto-[3,2-a]quinoline-3-carboxylate.
Mass analysis ($C_{24}H_{21}F_4N_3O_3S$) $M^+$: 507

Example 32. 6,8-difluoro-1-(3,4-difluorophenyl)-4-oxo-7-(1-piperazinyl)-4H-[1,3]thiazeto[3,2-a]quinoline-3-carboxylic acid.
Mass analysis ($C_{26}H_{15}F_4N_3O_3S$) $M^+$: 465

Example 33. Ethyl 6,8-difluoro-1-(3,4-difluorophenyl)-4-oxo-7-(1-piperazinyl)-4H-[1,3]thiazeto[3,2-a]-quinoline-3-carboxylate.
Mass analysis ($C_{28}H_{19}F_4N_3O_3S$) $M^+$: 493

Example 34. 6,8-difluoro-1-(3,4-difluorophenyl)-7-(4-methyl-1-piperazinyl)-4-oxo-4H-[1,3]thiazeto[3,2-a]-quinoline-3-carboxylic acid.
Mass analysis ($C_{22}H_{17}F_4N_3O_3S$) $M^+$: 479

Example 35. Ethyl 6,6-difluoro-1-(3,4-difluorophenyl)-7-(4-methyl-1-piperazinyl)-4-oxo-4H-[1,3]thiazeto-[3,2-a]quinoline-3-carboxylate.
Mass analysis ($C_{24}H_{21}F_4N_3O_3S$) $M^+$: 507

Example 36. 6,8-difluoro-1-methyl-7-morpholino-4-oxo-4H-[1,3]thiazeto[3,2-a]quinoline-3-carboxylic acid.
Mass analysis $M^+$: 368

Example 37. Ethyl 6,8-difluoro-1-methyl-7-morpholino-4-oxo-4H-[1,3]thiazeto [3,2-a]quinoline-3-carboxylate
Mass analysis $M^+$: 396

Example 38. 6,8 -difluoro-1-methyl-7-thiomorpholino-4-oxo-4H-[1,3] thiazeto[3,2-a]quinoline-3-carboxylic acid.
Mass analysis $M^+$: 384

Example 39. Ethyl 6,8-difluoro-1-methyl-7-thiomorpholino-4-oxo-4H-[1,3]thiazeto[3,2-a]quinoline-3-carboxylate.
Mass analysis $M^+$:412

Melting points and analytical data of several of the above-identified compounds are given in the following table.

| Example Number | Melting Point ("d" means decompn.) | Molecular Formula | Elementary Analysis C (upper column: calcd %) (lower column: found %) | H | N** |
|---|---|---|---|---|---|
| 5 | >300°C | $C_{15}H_{13}F_2N_3O_3S.H_2O$ | 48.51<br>48.75 | 4.07<br>4.11 | 11.31<br>11.07 |
| 6 | 220-230°C(d) | $C_{17}H_{17}F_2N_3O_3S.\frac{1}{2}H_2O$ | 52.30<br>52.42 | 4.65<br>4.59 | 10.76<br>10.68 |
| 7 | 239-252°C(d) | $C_{16}H_{15}F_2N_3O_3S.\frac{1}{2}H_2O$ | 51.06<br>51.23 | 4.28<br>4.01 | 11.16<br>10.96 |
| 8 | 227-230°C(d) | $C_{18}H_{19}F_2N_3O_3S$ | 54.67<br>54.54 | 4.84<br>4.97 | 10.63<br>10.59 |
| 9 | 250°C(d) | $C_{15}H_{13}F_2N_3O_3S.\frac{1}{2}H_2O$ | 49.72<br>49.57 | 3.89<br>3.72 | 11.60<br>11.24 |
| 10 | 300°C(d) | $C_{17}H_{17}F_2N_3O_3S.H_2O$ | 51.12<br>50.70 | 4.79<br>4.35 | 10.52<br>10.50 |
| 13 | 300°C(d) | $C_{16}H_{15}F_2N_3O_3S.2H_2O$ | 47.64<br>47.42 | 4.75<br>4.37 | 10.42<br>10.22 |
| 14 | 162°C(d) | | | | |
| 15 | 223-224°C(d) | $C_{17}H_{17}F_2N_3O_3S.\frac{1}{2}H_2O$ | 52.30<br>52.46 | 4.64<br>4.89 | 10.52<br>10.72 |
| 16 | 229-230°C(d) | $C_{19}H_{21}F_2N_3O_3S$ | 55.74<br>55.70 | 5.17<br>4.87 | 10.26<br>10.23 |
| 21 | 260-264°C(d) | $C_{21}H_{17}F_2N_3O_3S.\frac{1}{4}H_2O$ | 58.13<br>58.00 | 4.06<br>4.03 | 9.68<br>9.95 |
| 22 | 236-239°C(d) | $C_{23}H_{21}F_2N_3O_3S.\frac{1}{4}H_2O$ | 59.80<br>59.79 | 4.69<br>4.75 | 9.10<br>9.05 |
| 23 | 188-190°C(d) | $C_{22}H_{19}F_2N_3O_3S.{}^1/_5H_2O$ | 59.11<br>59.10 | 4.37<br>4.47 | 9.40<br>9.28 |

| | | | | | |
|---|---|---|---|---|---|
| 24 | 216-218°C(d) | $C_{24}H_{23}F_2N_3O_3S\cdot\frac{1}{4}H_2O$ | 60.56<br>60.77 | 4.98<br>5.12 | 8.83<br>8.71 |
| 24a | >300°C | $C_{21}H_{16}F_3N_3O_3S\cdot\frac{1}{4}H_2O$ | 55.26<br>55.19 | 3.75<br>3.56 | 9.21<br>9.38 |
| 25 | 159-163°C(d) | $C_{23}H_{20}F_3N_3O_3S\cdot\frac{1}{10}H_2O$ | 57.88<br>57.83 | 4.26<br>4.31 | 8.80<br>8.66 |
| 26 | 268-271°C(d) | $C_{22}H_{18}F_3N_3O_3S\cdot\frac{1}{10}H_2O$ | 54.90<br>54.98 | 4.23<br>4.03 | 8.73<br>8.85 |
| 27 | 165-167°C(d) | $C_{24}H_{22}F_3N_3O_3S$ | 58.89<br>58.69 | 4.53<br>4.59 | 8.58<br>8.57 |
| 28 | >300°C | $C_{21}H_{15}F_4N_3O_3S\cdot\frac{1}{2}H_2O$ | 53.17<br>53.19 | 3.40<br>3.32 | 8.86<br>8.76 |
| 29 | 285°C(d) | $C_{23}H_{19}F_4N_3O_3S\cdot\frac{1}{2}H_2O$ | 54.98<br>54.94 | 4.01<br>3.87 | 8.36<br>8.39 |
| 30 | 204-206°C(d) | $C_{22}H_{17}F_4N_3O_3S\cdot\frac{1}{4}H_2O$ | 54.60<br>54.64 | 3.64<br>3.58 | 8.68<br>8.65 |
| 31 | 185-187°C | $C_{24}H_{21}F_4N_3O_3S$ | 56.80<br>56.83 | 4.17<br>4.25 | 8.28<br>8.38 |
| 32 | >300°C | $C_{21}H_{15}F_4N_3O_3S\cdot\frac{1}{2}H_2O$ | 53.17<br>53.11 | 3.40<br>3.40 | 8.86<br>9.03 |
| 33 | 278°C(d) | $C_{23}H_{19}F_4N_3O_3S\cdot\frac{1}{10}H_2O$ | 55.78<br>55.69 | 3.91<br>3.84 | 8.48<br>8.51 |
| 34 | 230-233°C(d) | $C_{22}H_{17}F_4N_3O_3S$ | 55.11<br>54.89 | 3.57<br>3.64 | 8.76<br>8.60 |
| 35 | 190-192°C | $C_{24}H_{21}F_4N_3O_3S\cdot\frac{1}{10}H_2O$ | 56.60<br>56.52 | 4.20<br>4.10 | 8.25<br>8.12 |
| 36 | 245-246°C(d) | $C_{16}H_{14}F_2N_2O_4S$ | 52.17<br>52.22 | 3.83<br>3.72 | 7.61<br>7.59 |
| 37 | 185-189°C | $C_{18}H_{18}F_2N_2O_4S$ | 54.54<br>54.38 | 4.58<br>4.53 | 7.07<br>7.07 |
| 38 | 257°C(d) | $C_{16}H_{14}F_2N_3O_2S_2$ | 50.00<br>49.90 | 3.67<br>3.70 | 7.29<br>7.32 |
| 39 | 188-190°C | $C_{18}H_{18}F_2N_2O_3S_2$ | 52.42<br>52.32 | 4.40<br>4.51 | 6.79<br>6.71 |

Test Example.

As hereunder, result of pharmacological test showing the usefulness of the representative compounds of the present invention is given.

1. Measurement of minimum growth-inhibition concentrations (MIC).

Test Method: In accordance with a standard method by Japan Chemotherapeutic Society [Chemotherapy, 29(1), pages 76-79, 1981], agar plate dilution method was used and the MIC was measured. Thus, bouillon for measuring sensitivity was used and the bacterial liquid cultured at 37° C for 18 hours was diluted to an extent of $10^6$ CFU/ml using said medium. This was inoculated to an agar medium containing drug for measuring sensitivity using a microplanter, cultured at 37° C for 18 hours, and MIC was measured. Ofloxacin was used for comparison/control. The result is given in Table 1. It is apparent that the present invention compounds exhibit strong antibacterial activity against Pseudomonas aeruginosa and both gram-positive and negative bacteria.

Table 1

| | M I C (μg/ml) | |
|---|---|---|
| | The Present Invention Compd | Comparison/Control |
| Staphylococcus aureus 209-P JC-1 | 0.1 | 0.39 |
| Streptococcus pyogenes S-23 | 0.39 | 1.56 |
| Streptococcus pneumoniae Type I | 0.39 | 1.56 |
| Bacillus subtilis ATCC 6633 | 0.05 | 0.1 |
| Escherichia coli NIHJ JC-2 | 0.0125 | 0.1 |
| Klebsiella pneumoniae NCTC 9632 | 0.0125 | 0.05 |
| Serratia marcescens IFO 3736 | 0.1 | 0.78 |
| Proteins mirabilis IFO 3849 | 0.025 | 0.39 |
| Shigella flexneri 2a EW-10 | ≦0.00625 | 0.025 |
| Pseudomonas aeruginosa IFO-3445 | 0.1 | 1.56 |
| "The Present Invention Compd" and "Comparison/Control" mean the compound of Example 2 and ofloxacin, respectively. | | |

The test was conducted for some more compounds using the same microorganisms and the result is given in Table 2a.

Table 2a

| (MIC in μg/ml) | | | | | | |
|---|---|---|---|---|---|---|
| Tested Compounds: | Ex.4 | Ex.5 | Ex.7 | Ex.9 | Ex.21 | Ex.25 |
| | 0.2 | 0.1 | 0.2 | 0.025 | 0.2 | 0.2 |
| | 0.39 | - | - | - | - | - |
| | 0.39 | - | - | - | - | - |
| | 0.05 | 0.1 | 0.05 | 0.1 | 0.1 | 0.1 |
| | 0.05 | 0.025 | 0.05 | 0.0125 | 0.1 | 0.1 |
| | 0.025 | 0.025 | 0.0125 | 0.0125 | ≦0.00625 | ≦0.0625 |
| | 0.2 | 0.05 | 0.1 | 0.1 | 1.56 | 1.56 |
| | - | - | - | - | - | - |
| | 0.00625 | 0.0125 | 0.0125 | ≦0.00625 | 0.05 | 0.05 |
| | 0.2 | 0.2 | 0.39 | 0.0125 | 0.39 | 0.39 |

2. Therapeutic effect to infection in mice.

Test method: E. coli KC-14 and P. aeruginosa E-2 were suspended in 5% mucin and 0.5 ml of the suspension was injected intraperitoneally to ddY strain male mice (body weight: ca. 20 g; four weeks age; 10 mice per group). The amount of the bacteria inoculated was 5.1 x $10^4$ CFU/mouse for E. coli and 7.5 x

$10^4$ CFU/mouse for P. aeruginosa. Drug was given orally once after 2 hours of inoculation and, out of the survival rate after one week, $ED_{50}$ was calculated by a Probit method. As to a comparison/control, ofloxacin was used. The result is given in Table 2.

Table 2

| Compound (Example No.) | E $D_{50}$ (mg/mouse) | |
|---|---|---|
| | E. coli | P. aeruginosa |
| 1<br>2 | 0.0046<br>0.0078 | 0.09<br>0.154 |
| Ofloxacin | 0.011 | 0.692 |

Similar test was conducted by inoculating E. coli KC-14 (2.5 x $10^5$ cfu/mouse) and P. aeruginosa E-2 (1.25 x $10^5$ cfu/mouse) and the result is given in Table 3.

Table 3

| $ED_{50}$ (mg/mouse) | | | | |
|---|---|---|---|---|
| | The Present Invention Compd. | | | Ofloxacin |
| | Ex.2 | Ex.4 | Ex.7 | |
| E. coli KC-14<br>P. Aeruginosa E-2 | 0.013<br>0.156 | 0.011<br>0.177 | 0.014<br>0.263 | 0.052<br>0.521 |

It is apparent that the present invention compounds exhibit strong therapeutic effect to infectious diseases to mice.

(Effect)

It is obvious from the above facts and results that the compounds of the present invention are effective at far less doses than the conventional antibacterial agents not only to P. aeruginosa but also both gram-positive and negative bacteria and they exhibit wide antibacterial spectrum.

Furthermore the compounds of the present invention are very little toxic and, accordingly, they can be applied, with high safety, as therapeutic agents to systemic infectious diseases and local ones such as infectious diseases in urinary gall tracts of mammals including human being.

## Claims

**1.** Derivative of quinolinecarboxylic acid represented by the following general formula (I) and physiologically-acceptable salt thereof:

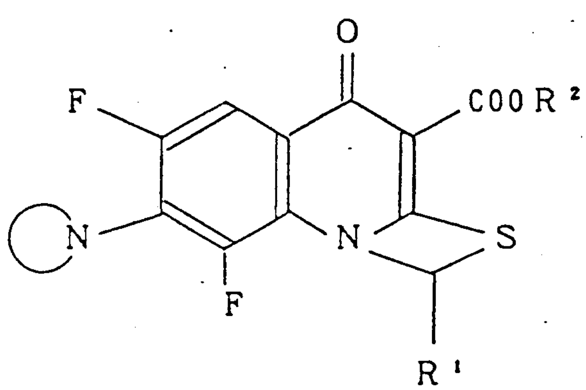

( I )

in which $R^1$ is hydrogen, straight chain or branched alkyl having 1 to 4 carbon atoms, unsubstituted phenyl or halogen-substituted phenyl; $R^2$ is hydrogen or straight chain or branched alkyl having 1 to 4 carbon atoms; and

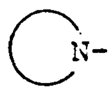

is five to six membered cyclic amino group which may be substituted with alkyl having 1 to 4 carbon atoms, amino or amino substituted with alkyl having 1 to 4 carbon atoms and may further contain nitrogen, oxygen or sulfur atom.

## Revendications

**1.** Dérivé de l'acide quinoléinecarboxylique représenté par la formule générale (I) suivante et ses sels physiologiquement acceptables :

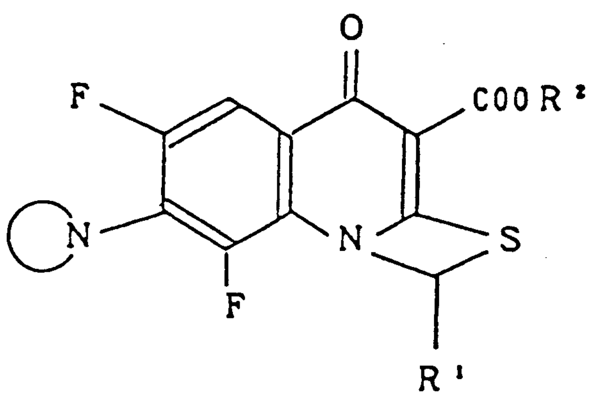

(I)

dans laquelle $R^1$ est un atome d'hydrogène, un groupe alkyle à chaîne linéaire ou ramifiée en $C_1$ à $C_4$, un groupe phényle non substitué ou substitué par un atome d'halogène; $R^2$ est un atome d'hydrogène, un groupe alkyle à chaîne linéaire ou ramifiée en $C_1$ à $C_4$; et

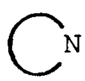

est un groupe amino cyclique à 5 à 6 maillons qui peut être substitué par un groupe alkyle en $C_1$ à $C_4$, amino ou amino substitué par un groupe alkyle en $C_1$ à $C_4$ et peut en outre contenir un atome d'azote, d'oxygène ou de soufre.

## Patentansprüche

**1.** Chinolincarbonsäurederivat der nachfolgenden allgemeinen Formel (I) und dessen physiologisch annehmbare Salze:

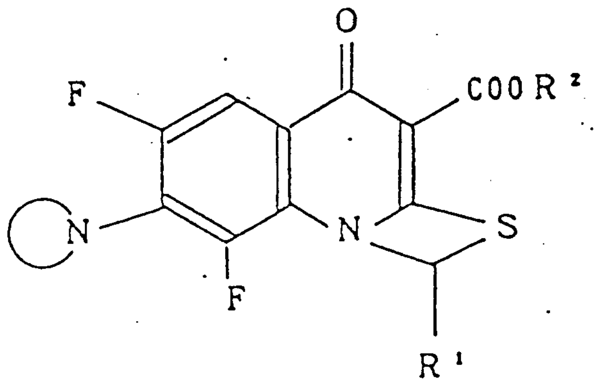

(I)

worin $R^1$ Wasserstoff, geradkettiges oder verzweigtes Alkyl mit 1 bis 4 Kohlenstoffatomen, unsubstituiertes Phenyl oder halogensubstituiertes Phenyl ist; $R^2$ Wasserstoff oder geradkettiges oder verzweigtes Alkyl mit 1 bis 4 Kohlenstoffatomen ist; und

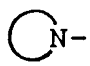

eine fünf- bis sechsgliedrige cyclische Aminogruppe ist, welche mit Alkyl mit 1 bis 4 Kohlenstoffatomen, mit Amino oder mit Amino, das mit Alkyl mit 1 bis 4 Kohlenstoffatomen substituiert ist, substituiert sein kann, und weiter Stickstoff-, Sauerstoff- oder Schwefelatome enthalten kann.